(19) Europäisches Patentamt
European Patent Office
Office européen des brevets

(11) Numéro de publication : **0 466 545 A1**

# (12) DEMANDE DE BREVET EUROPEEN

(21) Numéro de dépôt : **91401717.3**

(22) Date de dépôt : **26.06.91**

(51) Int. Cl.[5] : **C01B 33/20,** B01J 29/04, C07C 37/60, C07C 41/26

(30) Priorité : **29.06.90 FR 9008210**
**29.06.90 FR 9008209**
**31.08.90 FR 9010872**

(43) Date de publication de la demande :
**15.01.92 Bulletin 92/03**

(84) Etats contractants désignés :
**DE FR GB IT NL**

(71) Demandeur : **RHONE-POULENC CHIMIE**
**25, quai Paul Doumer**
**F-92408 Courbevoie Cédex (FR)**

(72) Inventeur : **Costantini, Michel**
**10, rue du Dr. Bonhomme**
**F-69003 Lyon (FR)**
Inventeur : **Guth, Jean-Louis**
**59, rue Bellevue**
**F-68200 Brunstatt (FR)**
Inventeur : **Lopez, Annie**
**Route Caillouville**
**F-76490 St. Wandrille Rançon (FR)**
Inventeur : **Popa, Jean-Michel**
**2, rue Roger Breton**
**F-93700 Drancy (FR)**

(74) Mandataire : **Dutruc-Rosset, Marie-Claude et al**
**RHONE-POULENC CHIMIE Service Brevets Chimie 25, Quai Paul Doumer**
**F-92408 Courbevoie Cédex (FR)**

## (54) Zéolithes à base de silice et d'oxydes d'éléments tétravalents, leur procédé de synthèse et leur application.

(57) La présente invention concerne des zéolithes à base de silice et d'oxydes d'éléments tétravalents choisis parmi l'étain et le zirconium.

L'invention a pour objet une zéolithe à base de silice et d'un oxyde d'élément tétravalent caractérisée par le fait qu'elle a la formule générale suivante (I), après calcination :

$$(Si_{91-x}\ T'_{x})\ O_{192} \qquad (I)$$

dans ladite formule (I) :

— x est compris entre 0,1 et 5,0,

— T′ symbolise un élément tétravalent choisi dans le groupe formé par l'étain et le zirconium.

Ces zéolithes ont une structure de type MFI et peuvent être utilisées notamment dans le domaine de la catalyse et plus particulièrement, dans un procédé d'hydroxylation de phénols ou d'éthers de phénols par le peroxyde d'hydrogène.

EP 0 466 545 A1

La présente invention concerne des zéolithes à base de silice et d'oxydes d'éléments tétravalents choisis parmi l'étain et le zirconium.

Plus précisément, l'invention a trait à des zéolithes à base de silice et d'oxyde d'étain et des zéolithes à base de silice et d'oxyde de zirconium.

L'invention concerne plus particulièrement des zéolithes de structure MFI, et leur procédé de synthèse.

L'invention vise également une de leurs applications telles que leur mise en oeuvre dans un procédé d'hydroxylation de phénols ou d'éthers de phénols par le peroxyde d'hydrogène.

Les zéolithes sont des tectosilicates cristallisés. Les structures sont constituées par des assemblages de tétraèdres $TO_4$ formant une charpente tridimensionnelle par la mise en commun des oxygènes. Dans les zéolithes du type aluminosilicate qui sont les plus communes, T représente le silicium tétravalent ainsi que l'aluminium trivalent. Les cavités et canaux de dimensions moléculaires de cette charpente accueillent les cations compensant le déficit de charge lié à la présence de l'aluminium trivalent dans les tétraèdres. Des éléments trivalents comme le gallium et plus rarement le bore ou le béryllium peuvent se substituer à l'aluminium.

D'une manière générale, la composition des zéolithes peut être représentée par la formule brute $M_{2}/_{n}O$ ; $Y_2O_3$ ; $xZO_2$ à l'état déshydraté et calciné. Z et Y représentent respectivement les éléments tétravalent et trivalent des tétraèdres $TO_4$ ; M représente un élément électropositif de valence n tel qu'un alcalin ou alcalinoterreux, constituant les cations de compensation ; x peut varier de 2 à théoriquement l'infini auquel cas la zéolithe est une silice.

Chaque type de zéolithe possède une structure poreuse distincte. La variation des dimensions et formes des pores d'un type à l'autre, entraîne des changements des propriétés adsorbantes. Seules les molécules ayant certaines dimensions et formes sont capables d'entrer dans les pores d'une zéolithe particulière. En raison de ces caractéristiques remarquables les zéolithes conviennent tout particulièrement pour la purification ou la séparation de mélanges gazeux ou liquides comme par exemple la séparation d'hydrocarbures par adsorption sélective.

La composition chimique avec en particulier la nature des éléments présents dans les tétraèdres $TO_4$ et la nature des cations de compensation échangeable, est également un facteur important intervenant dans la sélectivité de l'adsorption et surtout dans les propriétés catalytiques de ces produits. Ils sont utilisés comme catalyseurs ou supports de catalyseurs dans le craquage, le reformage et la modification d'hydrocarbures ainsi que dans l'élaboration de nombreuses molécules.

De nombreuses zéolithes existent dans la nature : ce sont des aluminosilicates dont les disponibilités et propriétés ne répondent pas toujours aux exigences des applications industrielles. De ce fait, la recherche de produits ayant des propriétés nouvelles a conduit à la synthèse d'une grande variété de zéolithes essentiellement du type aluminosilicate. Parmi les nombreux exemples de ce type on peut signaler : la zéolithe A (US-A 2882243), la zéolithe X (US-A 2882244), la zéolithe Y (US-A 3130007), la zéolithe L (FR-A 1224154), la zéolithe T (FR-A 1223775), la zéolithe ZSM5 (US-A 3702886), la zéolithe ZSM12 (US-A 3832449), la zéolithe ZSM48 (EP-A 0015132).

Les zéolithes sont généralement obtenues à partir d'un mélange réactionnel qui se transforme, en milieu hydrothermal, par un procédé de dissolution-recristallisation, le précipité cristallin étant, après séparation et séchage, calciné pour donner une zéolithe active.

Le mélange réactionnel contient des réactifs pouvant fournir les éléments T à incorporer dans la charpente de la zéolithe, ces réactifs sont généralement des gels aqueux contenant des oxydes ou hydroxydes des éléments T.

Il contient également un ou plusieurs mobilisateurs favorisant la dissolution de ces réactifs et le transfert depuis la phase aqueuse sur les cristaux de zéolithes en formation, et un agent structurant permettant, par son incorporation, la formation d'espaces microporeux ainsi qu'une stabilisation de la zéolithe.

Comme mobilisateurs, les anions $OH^-$ sont utilisés. Ainsi, les milieux réactionnels sont alors caractérisés par un pH basique généralement supérieur à 10. Ces milieux conviennent bien pour la dissolution des sources contenant les éléments silicium et aluminium, et d'une façon générale tous les éléments donnant des anions oxygénés solubles en milieu basique.

Lorsqu'on utilise des bases fortes tels les hydroxydes de métaux alcalins, on obtient des milieux très sursaturés permettant une cristallisation rapide des zéolithes. Mais il est souvent difficile de contrôler la formation de la phase cristallisée souhaitée qui est, dans beaucoup de cas, métastable. D'autre part, une vitesse de cristallisation élevée peut conduire à la formation de défauts dans la charpente de tétraèdres $TO_4$, tels que - T-$O^-$ à la place des pontages - T-O-T -. Enfin, la présence de cations de compensation alcalins dans les canaux et cavités est souvent gênante dans certaines applications et il peut alors être nécessaire de procéder à un échange avec d'autres cations. On peut éviter ce dernier inconvénient en remplaçant ces bases par d'autres bases fortes tels les hydroxydes d'alkylammonium qui peuvent jouer en même temps un rôle de structurant. Mais le prix élevé de ces produits limite leur utilisation industrielle. La mise en oeuvre de bases plus faibles

telles que les amines qui ont également des propriétés structurantes, élimine en grande partie les inconvénients cités. Mais dans ce cas, la concentration en anions $OH^-$ mobilisateurs peut devenir trop faible ce qui conduit à des vitesses de réaction trop faibles.

De plus, il n'est pas toujours facile d'incorporer certaines espèces métalliques dans les tétraèdres $TO_4$ de la charpente de la zéolithe quand le milieu réactionnel est basique.

Un objectif de la présente invention est de fournir une nouvelle zéolithe à base de silice et d'un oxyde d'élément tétravalent et plus précisément, une zéolithe à base de silice et d'oxyde d'étain et une zéolithe à base de silice et d'oxyde de zirconium.

Un autre objectif de l'invention est de les synthétiser selon un procédé évitant les inconvénients précités, à partir de mélanges réactionnels neutres ou acides permettant l'incorporation d'étain et/ou de zirconium dans la charpente de la zéolithe et conduisant à des cristaux de zéolithe de dimensions totalement contrôlées.

L'invention a donc pour objet des zéolithes à base de silice et d'oxydes d'éléments tétravalents ayant après calcination la formule générale (I) suivante:

$$(Si_{96-x}T'_x)\ O_{192} \qquad (I)$$

dans ladite formule (I):

– x compris entre 0,1 et 5,0,

– T′ symbolise un élément tétravalent choisi dans le groupe formé par l'étain et le zirconium.

Selon une nouvelle caractéristique de l'invention, les zéolithes appartiennent à la famille des pentasils et s'apparentent aux zéolithes du type structural MFI.

Selon une autre caractéristique de l'invention, les zéolithes contiennent du fluor. La concentration en fluor est généralement comprise entre 0,01 et 1,3 % en poids, avant calcination.

Les zéolithes à base de silice et d'oxyde d'étain, appelées stannozéosilites répondent préférentiellement à la formule donnée ci-après (Ia):

$$(Si_{96-x}\ Sn_x)\ O_{192} \qquad (Ia)$$

dans ladite formule (Ia):

– x est compris entre 0,1 et 3,0 environ, de préférence, entre 0,1 et 2,0.

Les zéolithes à base de silice et d'oxyde de ziconium, appelées zirconozéosilites répondent préférentiellement à la formule donnée ci-après (Ib):

$$(Si_{96-x}\ Zr_x)\ O_{192} \qquad (Ib)$$

dans ladite formule (Ib):

– x est compris entre 0,1 et 4,0 environ, de préférence, entre 0,1 et 2,0.

L'invention a également pour objet un procédé de synthèse des zéolithes conformes à l'invention ; ce procédé comporte:

(i) la préparation d'un mélange réactionnel en milieux aqueux contenant une source de silicium au degré d'oxydation + 4, une source d'élément tétravalent T′, des ions fluorure et un agent structurant ; le pH de ce mélange réactionnel étant compris entre 1,5 environ et 12,0 environ,

(ii) la cristallisation de ce mélange réactionnel par chauffage et la récupération du précipité cristallisé,

(iii) la calcination de celui-ci à une température supérieure à 450°C pour l'élimination de l'agent structurant occlus dans les canaux.

Avantageusement, le pH du mélange réactionnel est compris entre 6,0 et 11,0.

L'emploi d'ions fluorure dans le milieu réactionnel, qui jouent le rôle d'agent mobilisateur, permet d'obtenir une solubilisation des espèces T (Si et Sn ou Zr) dans un milieu de synthèse ayant un pH inférieur à 10. Ainsi, il est possible d'utiliser comme cations de compensation des ions $NH_4^+$, qui pourront être éliminés totalement, si on le désire, lors de la calcination. Par contre, il est conseillé d'éviter la présence, dans le milieu réactionnel de cations alcalins.

De plus, la cristallisation s'effectuant dans un milieu à pH inférieur à 11, la vitesse de nucléation est plus lente. Ainsi, il est possible d'obtenir des cristaux de zéolithes contrôlés par pilotage de la vitesse de nucléation.

De nombreuses sources de l'élément silicium au degré d'oxydation +4 peuvent être utilisées. On peut citer à titre d'exemple, les silices sous forme d'hydrogels, d'aérogels, de xérogels, de suspensions colloïdales, les silices résultant de la précipitation à partir de solutions de silicates solubles ou de l'hydrolyse d'esters siliciques comme $Si(OCH_3)_4$, $Si(OC_2H_5)_4$, les silices préparées par des traitements d'extraction et d'activation de composés cristallisés ou amorphes naturels ou synthétiques comme les silicates d'aluminium, les aluminosilicates, les argiles. On peut également utiliser des composés du silicium tétravalent hydrolysables tels les halogénures de silicium ou analogues.

En ce qui conerne les sources des éléments tétravalents T′, on peut faire appel plus particulièrement aux sources d'oxyde d'étain et de zirconium mentionnés ci-après.

Parmi les sources d'oxyde d'étain, on peut citer, à titre d'exemples, les oxydes et les hydroxydes d'étain, cristallisés ou amorphes, les composés d'étain tétravalent pouvant être hydrolysés tels les halogénures

($SnCl_4$), les alcoolates, les sels solubles d'étain tels le nitrate d'étain $Sn(NO_3)_4$.

Il est également possible d'utiliser comme sources de silice ou d'oxyde d'étain, des composés comprenant les éléments Si et Sn tels que, par exemple, des verres ou gels à base des oxydes de ces deux éléments.

Parmi les sources d'oxyde de zirconium, on peut citer, à titre d'exemples, les oxydes et les hydroxydes de zirconium, cristallisés ou amorphes, les composés de zirconium tétravalent pouvant être hydrolysés tels les halogénures ($ZrCl_4$), les alcoolates, les sels solubles de zirconium tels que le nitrate de zirconium $Zr(NO_3)_4$ ou le sulfate de zirconium $Zr(SO_4)_2$,x$H_2$0.

On peut également utiliser comme sources de silice ou d'oxyde de zirconium, des composés comprenant les éléments Si et Zr tels que, par exemple, des verres ou gels à base des oxydes de ces deux éléments.

Les sources de silice et d'oxyde d'étain et/ou d'oxyde de zirconium peuvent être engagées sous forme soluble ou de solides pulvérulents mais également sous forme d'agglomérats tels que, par exemple, pastilles, extrudés pouvant être transformés en zéolithe de structure désirée sans modification de forme.

L'agent mobilisateur $F^-$ est introduit sous forme d'acide et/ou de sel(s), ne contenant pas de cations alcalins, et/ou de composés libérant $F^-$ par hydrolyse. On peut citer, à titre d'exemple, l'acide fluorhydrique ; les fluorhydrates d'amines ; les fluorures d'ammonium quaternaire, par exemple $NH(C_3H_7)_3F$, $N(C_3H_7)_4F$ ; $SiF_4$ ; $SnF_4$ ; $(NH_4)_2SiF_6$.

Le fluorure d'ammonium ou le fluorure d'ammonium acide sont des sels préférés. En effet, ces sels sont très solubles et n'apportent aucun élément indésirable et, de plus, ils sont facilement éliminables en fin de cristallisation.

Les agents structurants convenant pour la mise en oeuvre de l'invention sont :

– les amines tertiaires de formule (II) :

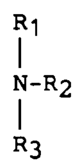

(II)

dans laquelle:

$R_1$, $R_2$ , $R_3$ identiques ou différents représentent un groupe alkyle, linéaire ou ramifié ayant de 1 à 6 atomes de carbone, de préférence un groupe propyle ou butyle.

– les sels d'ammonium quaternaire de formule (III):

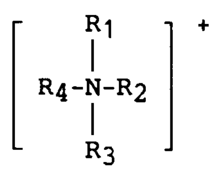

(III)

dans laquelle:

$R_1$, $R_2$ , $R_3$ , $R_4$ identiques ou différents représentent un groupe alkyle, linéaire ou ramifié ayant de 1 à 6 atomes de carbone, de préférence des groupes propyle ou butyle.

– les composés de formules (II) et (III) dans lesquelles l'azote a été remplacé par un atome de phosphore.

Selon une caractéristique préférée de l'invention, les agents structurants sont les composés qui peuvent fournir des cations tétrapropylammonium ou tripropylammonium.

Avantageusement, l'agent structurant est introduit dans le mélange réactionnel sous forme de sel d'ammonium quaternaire de formule (III) ou de l'amine de formule (II) ; le pH étant éventuellement ajusté à l'aide d'une base. De préférence, cette base aura des propriétés d'agent structurant faible pour ne pas concurrencer l'agent structurant ajouté. Ainsi, les bases convenables pour l'invention sont à titre d'exemple, la méthylamine, la diméthylamine, la triméthylamine, l'éthylamine, la diéthylamine et la triéthylamine.

Le mélange réactionnel a la composition suivante, exprimée en rapport molaire :

T'/(Si+T') compris entre 0,001 et 0,20, de préférence entre 0,005 et 0,1.

Agent structurant/(Si+T') compris entre 0,002 et 4, de préférence entre 0,04 et 1.

F/(Si+T') compris entre 0,02 et 6, de préférence entre 0,06 et 2.

$H_2O$/(Si+T') compris entre 4 et 400, de préférence 10 et 200.

Quand une base est utilisée pour ajuster le pH, le rapport molaire de la base par rapport à (T'+Si) est, de préférence, inférieur à 12, et de préférence compris entre 0,1 et 8.

L'ajout à ce mélange réactionnel, de germes cristallisés de structure déterminée, MFI, dans une proportion qui n'excède pas quelques pourcents pondéraux par rapport au poids de $SiO_2$ + $T'O_2$ engagé, facilite la cristallisation de la zéolithe. A titre de germes de cristallisation, on peut faire appel à toute zéolithe ayant la structure MFI, quelle que soit sa composition chimique. On peut utiliser de préférence, une zéosilite qui est une zéolithe équivalente à la stannozéosilite ou la zirconozéosilite, mais ne contenant que du silicium dans sa charpente. On peut également mettre en oeuvre, selon le cas, des germes de stannozéosilite ou de zirconozéosilite provenant d'une fabrication précédente.

La cristallisation de la zéolithe peut être obtenue par chauffage du mélange réactionnel à une température comprise entre 40°C environ et 240°C environ, de préférence entre 60°C et 220°C pendant le temps nécessaire à la cristallisation, selon un mode opératoire classique de synthèse de zéolithe et connu de l'homme du métier. A titre indicatif, la durée de chauffage peut être comprise entre 6 heures et 500 heures environ.

Ce chauffage et cette cristallisation sont réalisés de préférence dans un récipient ou autoclave revêtu d'une couche telle que, par exemple, le polytétrafluoroéthane.

Le mélange réactionnel peut être agité ou non.

Après cristallisation, le précipité obtenu est recueilli, par exemple, par filtration.

Ce précipité est ensuite chauffé après un séchage éventuel, à une température supérieure à 450°C, de préférence supérieure à 500°C, afin de décomposer par calcination ou décomposition thermique les espèces organiques contenues dans le précipité, telles que, par exemple, l'agent structurant.

On obtient ainsi selon que l'élément tétravalent est l'étain ou le zirconium, respectivement, une stannozéosilite ou une zirconozéosilite de l'invention de structure MFI.

Comme mentionné précédemment, les zéolithes contiennent du fluor, à raison de 0,01 et 1,3 % en poids. Ce fluor peut toutefois être éliminé par un traitement hydrothermal à $pH > 7$ sans pour autant modifier la structure de la zéolithe de l'invention.

L'invention a également pour objet les zéolithes obtenues avant calcination dénommées par la suite "précurseurs de zéolithes" qui sont des produits cristallins du type de zéolithes à base de silice et d'oxydes d'éléments tétravalents répondant à la formule (IV) suivante :

$$(Si_{96-x}\,T'_x)O_{192},\ 4\pm1(S^+F^-) \qquad (IV)$$

dans ladite formule (IV):

- x est compris entre 0,1 et 5,0,
- $S^+$ symbolise le cation provenant de l'agent structurant.
- T' symbolise un élément tétravalent choisi dans le groupe formé par l'étain et le zirconium.

Les précurseurs de zéolithes à base de silice et d'oxyde d'étain, répondent préférentiellement à la formule donnée ci-après (IVa)

$$(Si_{96-x}\,Sn_x)O_{192},\ 4\pm1(S^+F^-) \qquad (IVa)$$

dans ladite formule (IVa):

- x est compris entre 0,1 et 3,0 environ, de préférence, entre 0,1 et 2,0,
- $S^+$ symbolise le cation provenant de l'agent structurant.

Les précurseurs de zéolithes à base de silice et d'oxyde de ziconium répondant préférentiellement à la formule donnée ci-après (IVb):

$$(Si_{96-x}\,Zr_x)O_{192},\ 4\pm1(S^+F^-) \qquad (IVb)$$

dans ladite formule (IVb):

- x est compris entre 0,1 et 4,0 environ, de préférence, entre 0,1 et 2,0,
- $S^+$ symbolise le cation provenant de l'agent structurant.

$S^+$ représente plus particulièrement un cation résultant de l'amine de formule (II), un cation de type d'ammonium quaternaire de formule (III), ou ces mêmes composés dans lesquelles l'azote est remplacé par un atome de phosphore.

L'identification de la zéolithe à structure MFI peut être notamment réalisée par l'établissement du diagramme de diffraction du précurseur gui lui donne naissance.

Les précurseurs de zéolithes de l'invention ont un système cristallin orthorhombique et un diagramme de diffraction des rayons X définis dans le tableau I pour le précurseur de zéolithe à base de silice et d'oxyde d'étain et pour le précurseur de zéolithe à base d'oxyde de zirconium.

Dans ce tableau, les valeurs extrêmes des différentes équidistances réticulaires $d_{hkl}$ sont données et correspondent aux concentrations limites de l'élément tétravalent incorporé dans la charpente de la zéolithe avant calcination, ou plus précisément au rapport T'/(Si + T').

Ce diagramme de diffraction peut être obtenu à l'aide d'un diffractomètre en utilisant la méthode classique des poudres avec le rayonnement $K\alpha$ du cuivre. A partir de la position des pics de diffraction représentée par l'angle $2\theta$ on calcule, par la relation de Bragg, les équidistances réticulaires $d_{hkl}$ caractéristiques de l'échantillon. L'estimation de l'erreur de mesure $\Delta(d_{hkl})$ sur $d_{hkl}$ se calcule, en fonction de l'erreur absolue $\Delta(2\theta)$ affectée à

la mesure de 2θ, par la relation de Bragg. Une erreur absolue Δ(2θ) égale à ± 0,2° est couramment admise. L'intensité relative I/Io affectée à chaque valeur de $d_{hkl}$ est estimée à partir de la hauteur du pic de diffraction correspondant. On utilise souvent une échelle de symboles pour caractériser cette intensité : FF = très fort, F = fort, mF = moyen à fort, m = moyen, mf = moyen à faible, f = faible, ff = très faible.

La valeur du volume $V_o$ de la maille cristallographique de la zéolithe avant calcination est fonction de la substitution du silicium par l'élément tétravalent.

Les précurseurs des zéolithes de l'invention ont un diagramme de diffraction des rayons X, dont les principales raies sont indiquées dans le tableau I.

TABLEAU I

Diagramme de diffraction des rayons X

| Valeurs extrêmes des $d_{hkl}$ (nm) | $I/I_o$ | Valeurs extrêmes des $d_{hkl}$ (nm) | $I/I_o$ |
|---|---|---|---|
| 1,112-1,133 | F | 0,372-0,379 | mF |
| 0,988-1,014 | F | 0,368-0,375 | mF |
| 0,969-0,983 | mF | 0,361-0,368 | mF |
| 0,886-0,908 | f | 0,356-0,362 | f |
| 0,734-0,752 | m | 0,343-0,349 | f |
| 0,699-0,713 | f | 0,340-0,346 | m |
| 0,660-0,677 | f | 0,330-0,336 | m |
| 0,627-0,639 | m | 0,327-0,334 | m |
| 0,597-0,611 | m | 0,321-0,327 | mf |
| 0,591-0,603 | m | 0,300-0,306 | m |
| 0,564-0,576 | m | 0,294-0,299 | m |
| 0,548-0,562 | m | 0,291-0,296 | mf |
| 0,492-0,503 | mf | 0,257-0,262 | f |
| 0,492-0,503 | mf | 0,253-0,258 | f |
| 0,454-0,464 | mf | 0,248-0,252 | f |
| 0,432-0,440 | mf | 0,247-0,251 | f |
| 0,420-0,429 | m | 0,238-0,241 | f |
| 0,394-0,404 | mf | 0,237-0,240 | f |
| 0,390-0,397 | f | 0,198-0,202 | mf |
| 0,379-0,387 | FF | 0,197-0,201 | m |

Ledits précurseurs de zéolithes sont susceptibles d'être obtenus par le procédé comprenant :

(i) la préparation d'un mélange réactionnel en milieu aqueux contenant au moins une source de silicium au degré d'oxydation + 4, une source d'élément tétravalent, des ions fluorure, un agent structurant ; le pH du mélange réactionnel étant compris entre 1,5 et 12,0, de préférence entre 6,0 et 11,0.

(ii) la cristallisation du mélange réactionnel et la récupération du précipité cristallin.

Les rapports molaires des différentes espèces dans le milieu réactionnel sont ceux indiqués précédemment.

Le précipité cristallin est avantageusement lavé pour éliminer les impuretés et notamment les cations ou anions non accrochés ou incorporés dans la structure.

Ce produit manipulable est notamment et principalement utilisé pour la production de zéolithe par calcination sous des conditions appropriées et déterminées en fonction de l'utilisation désirée de la zéolithe.

Les zéolithes de l'invention peuvent être utilisées dans la catalyse de nombreuses réactions telles que, par exemple, les réactions de dismutation ou d'alkylation, l'hydrogénolyse et l'hydrogénation de coupes pétrolières ou dans les procédés de reformage.

Un autre objet de la présence invention réside dans l'utilisation des zéolithes de l'invention comme catalyseurs de la réaction d'hydroxylation des phénols ou des éthers de phénols par le peroxyde d'hydrogène.

La présente invention vise donc également un procédé d'hydroxylation des phénols et des éthers de phénols utilisant les catalyseurs ci-dessus

L'invention concerne plus particulièrement les phénols ou éthers de phénols de formule générale (V):

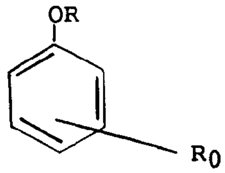

(V)

dans ladite formule (V):

– R représente un atome d'hydrogène, un groupement méthyle, un groupement éthyle ou un groupement phényle,

– $R_0$ représente un atome d'hydrogène, un radical alkyle ayant 1 à 4 atomes de carbone, un radical alkoxy ayant 1 à 4 atomes de carbone, un radical phényle ou cyclohexyle.

Conformément à la présente invention, on fait réagir le phénol ou l'éther de phénol de formule (V) avec le peroxyde d'hydrogène, en présence d'une quantité efficace d'une zéolithe de structure MFI à base de silice et d'un oxyde d'élément tétravalent de formule (I), et plus préférentiellement en présence d'une stannozéosilite de formule (Ia) ou ou d'une zirconozéosilite de formule (Ib).

Le peroxyde d'hydrogène peut être utilisé sous la forme d'une solution aqueuse ayant généralement une concentration en peroxyde d'hydrogène supérieure à 20 % en poids. Le peroxyde d'hydrogène peut également être utilisé sous la forme d'une solution dans un solvant organique. Comme solvants organiques utilisables pour la mise en oeuvre du peroxyde d'hydrogène, on peut utiliser des esters tels que notamment les esters d'alkyle ou de cycloalkyle d'acides carboxyliques aliphatiques saturés ; de préférence on emploiera les acétates et les propionates d'alkyle ayant au total de 4 à 8 atomes de carbone ou des mélanges de tels esters. On peut également utiliser des solutions de peroxyde d'hydrogène dans un éther tel que par exemple le dioxanne, le diisopropyléther ou le méthyl-tertiobutyléther.

Le rapport molaire composé de formule (V)/peroxyde d'hydrogène est généralement de 25/1 à 3/1 et préférentiellement de 20/1 à 4/1.

La quantité de stannozéosilite ou de zirconozéosilite, objet de la présente invention, que l'on peut mettre en oeuvre dans le présent procédé peut varier dans de très larges limites.

Lorsque l'on réalise le procédé en discontinu, le catalyseur peut représenter en poids par rapport au poids du composé de formule (V) engagé de 0,1 % à 20 %. De préférence, ce rapport pondéral sera compris entre 0,5 % et 10 %. Cependant si l'on réalise le procédé en continu, par exemple en faisant réagir un mélange de composé (V) et de solution de peroxyde d'hydrogène sur un lit fixe de catalyseur, ces rapports catalyseur-/composé (V) n'ont plus de sens et, à un instant donné, on pourra avoir un excès pondéral de catalyseur par rapport au composé (V).

Il est également possible d'effectuer la réaction d'hydroxylation du composé (V) dans un solvant du composé (V), qui soit de préférence miscible ou partiellement miscible à l'eau.

A titre d'exemples non limitatifs de tels solvants, on peut citer l'eau ; les alcools comme le méthanol, le tertiobutanol, l'isopropanol ou l'éthanol ; les cétones comme l'acétone ou la méthylisobutylcétone ; les nitriles comme l'acétonitrile ; les acides carboxyliques comme l'acide acétique ; les esters comme l'acétate de propyle ; les éthers comme le méthyl-tertiobutyléther ; des solvants aprotiques polaires comme le dioxyde de tétrahydrothiophène (sulfolane), le carbonate d'éthylène glycol, le carbonate de propylène glycol, la N-méthylpyrrolidone.

La température à laquelle est conduite la réaction est généralement comprise entre 45° et 160°C sous pression atmosphérique. On peut également opérer à plus haute température et sous pression supérieure à la pression atmosphérique.

Les phénols et éthers de phénols qui sont utilisés de préférence dans le procédé de l'invention sont les composés de formule (V) dans laquelle R représente un atome d'hydrogène, un groupement méthyle ou un groupement éthyle, et $R_0$ représente un atome d'hydrogène, un groupement méthyle, éthyle ou tertiobutyle, un groupement méthoxy ou éthoxy.

A titre d'exemples non limitatifs, on peut citer le phénol, l'anisole, l'orthocrésol, le métacrésol, le paracrésol, le tertiobutyl-4 phénol, le méthoxy-2 phénol, le méthoxy-4 phénol.

Le procédé selon l'invention s'applique particulièrement bien au phénol pour la préparation d'hydroquinone et de pyrocatéchine.

D'autres buts, caractéristiques et détails de l'invention apparaîtront plus clairement au vu des exemples suivants donnés uniquement à titre indicatif et illustratif.

Les exemples 1 à 3 ont trait à des stannozéosilites et leurs modes d'obtention.

L'exemple 4 est relatif à une zirconozéosilite et son procédé de préparation.

L'exemple 5 concerne la mise en oeuvre de la stannozéosilite de l'exemple 1 de l'invention, dans un procédé d'hydroxylation du phénol.

L'exemple 6 est aussi un exemple d'application relatif à la zirconozéosilite.

Exemple 1

Cet exemple décrit une synthèse d'une stannozéosilite en milieu acide avec utilisation des ions $F^-$, comme agent mobilisateur.

On ajoute goutte à goutte et sous forte agitation, 0,26 g de chlorure d'étain $SnCl_4$ à une solution contenant :

- 13,2 g de bromure de tétrapropylammonium (TPA-Br),
- 11 g de fluorure d'ammonium $NH_4F$,
- 120 g d'eau,
- 6 g de silice AEROSIL 130 commercialisée par la Société DEGUSSA.

Après homogénéisation du gel, on ajoute à titre de germes de cristallisation, 0,12 g d'une zéolithe de structure MFI, à savoir une zéosilite.

Le pH du mélange réactionnel est de 8,5.

Les rapports molaires dans le mélange réactionnel sont les suivants :

Sn/Si = 0,01 ; F/Si = 3,0 ; $TPA^+$/Si = 0,5 ; $H_2O$/Si = 70.

On transfère le mélange réactionnel dans un autoclave dont les parois sont gainées de polytétrafluoroéthane.

On chauffe pendant 1 jour à 200°C.

Le pH du milieu à l'ouverture de l'autoclave est de 9,0.

Après filtration, lavage à l'eau et séchage à 80°C, on obtient 6,6 g de précurseur de stannozéosilite de type MFI pure dont le diagramme de diffraction X est conforme à celui donné dans le tableau I.

Les cristaux obtenus sont des bâtonnets de dimensions 5 x 1 µm.

L'analyse chimique du produit donne une teneur en fluor de 1,0 %.

On calcine le précurseur précédemment obtenu, à 550°C pendant 6 heures, ce qui permet d'éliminer l'agent structurant et de restaurer la porosité de la stannozéosilite obtenue.

Exemple 2

On prépare un gel mixte contenant les éléments Si et Sn. A cet effet, on ajoute lentement sous agitation, 2,6 g de chlorure d'étain $SnCl_4$ dans 20 g de méthanol anhydre, puis 15,2 g de tétraméthylate de silicium $Si(OCH_3)_4$. Après homogénéisation, on ajoute dans ce mélange une solution contenant 15 g d'une solution aqueuse d'ammoniaque à 25 %, 6 g de méthanol et 10 g d'eau. La prise en masse du gel est instantanée. Il est ensuite redispersé dans de l'eau puis évaporé et séché à 120°C. La poudre obtenue est relavée à l'eau pour éliminer le chlorure d'ammonium éventuellement formé. On obtient ainsi environ 10 g d'un gel mixte ($SiO_2$, $SnO_2$) avec un rapport molaire Sn/Si = 0,1.

On disperse ce gel dans une solution contenant 1,85 g de fluorure d'ammonium de $NH_4F$, 3,32 g de TPABr, 36 g d'eau et 0,12 g d'une zéosilite, zéolithe à structure MFI comme germes de cristallisation.

Le pH du milieu réactionnel est de 7.

Les rapports molaires dans le mélange réactionnel sont :

Sn/Si = 0,1 ; F/Si = 0,5 ; TPA+/Si = 0,125 ; $H_2O$/Si = 20.

On chauffe ce gel 6 jours à 200°C.

Le pH du milieu à l'ouverture de l'autoclave est de 9,5.

Après filtration, lavage à l'eau et séchage, on obtient 8,6 g de solide dont l'analyse par diffraction aux rayons X révèle qu'il s'agit d'un précurseur de zéolithe du type MFI dont le diagramme est conforme à celui donné dans le tableau I et qu'il y a présence d'impureté cristallisée du type $SnO_2$ cassitérite (moins de 10 %).

Les cristaux obtenus sont des bâtonnets de dimension 8 x 2 µm.

Une analyse ponctuelle réalisée par microsonde électronique donne des rapports Si/Sn à l'intérieur des cristaux compris entre 20 et 60. L'analyse chimique du produit donne une teneur en fluor de 0,91 %.

Après calcination à 550°C pendant 6 heures, on obtient une stannozéosilite .

Exemple 3

On prépare un gel mixte contenant les éléments Si et Sn comme décrit dans l'exemple 2. On disperse ce gel dans une solution contenant 1,85 g de $NH_4F$, 6,65 g de TPABr et 72 g d'eau.

Le pH est de 7,5.

Les rapports molaires dans le mélange réactionnel sont :

$Sn/Si = 0,1$ ; $F/Si = 0,5$ ; $TPA^+/Si = 0,25$ ; $H_2O/Si = 40$.

On chauffe ce gel 6 jours à 200°C dans une étuve équipée d'un système d'agitation.

Le pH du milieu à l'ouverture de l'autoclave est de 9.

Après filtration, lavage et séchage, on obtient 8,9 g de solide dont l'analyse par diffraction aux rayons X révèle qu'il s'agit d'un précurseur de zéolithe du type MFI ayant un diagramme conforme à celui dessiné dans le Tableau I et qu'il y a présence d'une impureté cristallisée du type $SnO_2$ cassitérite.

Les cristaux obtenus ont une longueur de 1 à 10 μm pour une largeur de 0,5 à 2 μm.

Après calcination à 550°C pendant 6 heures, on obtient une stannozéosilite.

Exemple 4

Cet exemple décrit une synthèse d'une zirconozéosilite en milieu acide avec utilisation des ions $F^-$, comme agent mobilisateur.

On ajoute goutte à goutte et sous forte agitation, 0,23 g de chlorure de zirconium $ZrCl_4$ à une solution contenant :

- 13,2 g de bromure de tétrapropylammonium (TPA-Br),
- 11 g d'une solution aqueuse d'acide fluorhydrique à 40 % en poids,
- 75 g d'eau,
- 6 g de silice AEROSIL 130 commercialisée par la Société DEGUSSA.

Après homogénéisation, on ajoute 78 g d'une solution aqueuse de méthylamine à 40 % et à titre de germes de cristallisation, 0,12 g d'une zéolithe de structure MFI, à savoir une zéosilite.

Le pH du mélange réactionnel est de 11,0.

Les rapports molaires dans le mélange réactionnel sont les suivants:

$Zr/Si = 0,01$ ; $F/Si = 3,0$ ; $TPA^+/Si = 0,5$ ; $H_2O/Si = 70$ ;

$CH_3NH_2/Si = 10$.

On transfère le mélange réactionnel dans un autoclave dont les parois sont gainées de polytétrafluoroéthane.

On chauffe pendant 1 jour à 200°C.

Le pH du milieu à l'ouverture de l'autoclave est de 11,5.

Après filtration, lavage à l'eau et séchage à 80°C, on obtient 6,0 g de précurseur de zirconozéosilite de type MFI dont le diagramme de diffraction X est conforme à celui donné dans le tableau I.

Les cristaux obtenus sont des plaquettes de dimensions 5 x 2,5 μm.

Une analyse ponctuelle réalisée par microsonde électronique donne un rapport Si/Zr à l'intérieur des cristaux d'environ 140.

L'analyse chimique du produit donne une teneur en fluor de 1,0 %.

On calcine le précurseur précédemment obtenu, à 550°C pendant 6 heures ce qui permet d'éliminer l'agent structurant et de restaurer la porosité de la zirconozéosilite obtenue.

Exemple 5

Procédé d'hydroxylation du phénol:

Dans un réacteur en verre pyrex de 30 cm³, muni d'une agitation centrale par barreau aimanté, d'un réfrigérant relié à un gazomètre, d'un système de chauffage régulé et d'un système d'injection, on charge après avoir préalablement purgé l'appareil à l'aide d'azote :

- 9,4 g de phénol (0,10 mol)
- 0,25 g de stannozéosilite préparée dans l'exemple 1.

On chauffe à 80°C sous agitation, puis on injecte une solution aqueuse de $H_2O_2$ à 70 % en poids par volume. (0, 005 mol de $H_2O_2$).

On laisse ensuite réagir pendant 2 heures 30 minutes.

Après filtration du catalyseur, on dose $H_2O_2$ non transformée par iodométrie et les diphénols par chromatographie liquide haute performance (CLHP).

On obtient les résultats suivants:

- taux de transformation (TT)de $H_2O_2$ : 61,0 %
- rendement en pyrocatéchine par rapport à $H_2O_2$ transformée (RT) : 23,0 %
- rendement en hydroquinone par rapport à $H_2O_2$ transformée (RT) : 4,5 %
- rendement total en diphénols : 27,5 %

Exemple 6

Procédé d'hydroxylation du phénol:

On a reproduit à l'identique l'exemple précédent, à la différence près que l'on met en oeuvre la zirconozéosilite de l'exemple 5, à la place de la stannozéosilite. On obtient des résultats équivalents, soit un taux de transformation de $H_2O_2$ de 62,0 % et un rendement total en diphénols de 28 %.

## Revendications

**1.** Zéolithe à base de silice et d'un oxyde d'élément tétravalent caractérisée par le fait qu'elle a la formule générale suivante (I), après calcination :

$$(si_{96-x}\, T'_x)\, O_{192} \qquad (I)$$

dans ladite formule (I):
– x est compris entre 0,1 et 5,0,
– T' symbolise un élément tétravalent choisi dans le groupe formé par l'étain et le zirconium.

**2.** Zéolithe selon la revendication 1, caractérisée par le fait que la structure de la zéolithe est du type structure MFI.

**3.** Zéolithe selon la revendication 1 ou 2, caractérisée par le fait qu'elle contient entre 0,01 et 1,3 % en poids de fluor, avant calcination.

**4.** Zéolithe selon l'une des revendications 1 à 3, caractérisée par le fait qu'elle répond à la formule (Ia):

$$(Si_{96-x}\, Sn_x)\, O_{192} \qquad (Ia)$$

dans ladite formule (Ia):
– x est compris entre 0,1 et 3,0 environ, de préférence, entre 0,1 et 2,0.

**5.** Zéolithe selon l'une des revendications 1 à 3, caractérisée par le fait qu'elle répond à la formule (Ib):

$$(Si_{96-x}\, Zr_x)\, O_{192} \qquad (Ib)$$

dans ladite formule (Ib):
- x est compris entre 0,1 et 4,0 environ, de préférence, entre 0,1 et 2,0.

**6.** Procédé de fabrication d'une zéolithe selon l'une des revendications précédentes, caractérisé par le fait qu'il comprend les étapes suivantes :
(i) la préparation d'un mélange réactionnel en milieux aqueux contenant une source de silicium au degré d'oxydation + 4, une source d'élément tétravalent, des ions fluorure et un agent structurant avec des rapports molaires T'/(Si+T') compris entre 0,001 et 0,20, F/(Si+T') entre 0,02 et 6, agent structurant/(Si+T') entre 0,002 et 4 ; le pH de ce mélange réactionnel étant compris entre 1,5 environ et 12,0 environ, de préférence entre 6,0 et 11,0,
(ii) la cristallisation du mélange réactionnel,
(iii) la récupération et calcination du précipité cristallin à une température supérieure à 450°C.

**7.** Procédé selon la revendication 6, caractérisé par le fait que le rapport molaire T'/(Si+T') est compris entre 0,005 et 0,1.

**8.** Procédé selon l'une des revendications 6 et 7 caractérisé par le fait que le rapport molaire F/(Si+T') est

compris entre 0,06 et 2.

**9.** Procédé selon l'une des revendications 6 à 8 caractérisé par le fait que le rapport molaire agent structurant/(Si+T') est compris entre 0,04 et 1.

**10.** Procédé selon l'une des revendications 6 à 9, caractérisé par le fait que le rapport molaire $H_2O/(Si+T'))$ dans le milieu réactionnel est compris entre 4 et 400, de préférence entre 10 et 200.

**11.** Procédé selon l'une des revendications 6 à 10, caractérisé par le fait que le pH du milieu réactionnel est contrôlé par l'addition d'une base ne contenant pas d'ions alcalins.

**12.** Procédé selon la revendication 11, caractérisé par le fait que la base est choisie dans le groupe comprenant la méthylamine, la diméthylamine, la triméthylamine, l'éthylamine, la diéthylamine et la triéthylamine.

**13.** Procédé selon la revendication 11 ou 12, caractérisé par le fait que le rapport molaire base/(T'+Si) dans le milieu réactionnel est inférieur à 12, de préférence entre 0,1 et 8.

**14.** Procédé selon l'une des revendications 6 à 13, caractérisé par le fait que l'oxyde de silicium et l'oxyde d'élément tétravalent ont une source commune.

**15.** Procédé selon l'une des revendications 6 à 14, caractérisé par le fait que l'agent structurant précité est choisi dans le groupe comprenant :

– les amines tertiaires de formule (II):

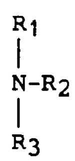

(II)

dans laquelle:

$R_1$ , $R_2$, $R_3$ identiques ou différents représentent un groupe alkyle, linéaire ou ramifié ayant de 1 à 6 atomes de carbone, de préférence un groupe propyle ou butyle.

– les sels d'ammonium quaternaire de formule (III):

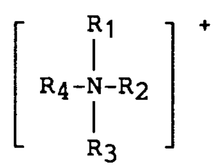

(III)

dans laquelle:

$R_1$ , $R_2$ , $R_3$ , $R_4$ identiques ou différents représentent un groupe alkyle, linéaire ou ramifié ayant de 1 à 6 atomes de carbone, de préférence des groupes propyle ou butyle.

– les composés de formules (II) et (III) dans lesquelles l'azote a été remplacé par un atome de phosphore.

**16.** Procédé selon l'une des revendications 6 à 13 et 15, caractérisé par le fait que la source d'oxyde de silicium est choisie dans le groupe comprenant les hydrogels, aérogels ou xérogels, suspensions colloïdales de silice, les esters siliciques, les silicates solubles dans l'eau, les silices extraites de composés cristallins naturels ou synthétiques, les composés du silicium tétravalent hydrolysables tels que les halogénures de silicium.

**17.** Procédé selon l'une des revendications 6 à 13, 15 et 16, caractérisé par le fait que la source d'étain est choisie dans le groupe comprenant les oxydes et les hydroxydes d'étain cristallisés ou amorphes, les halogènures, les alcoolates, les sels solubles d'étain et la source de zirconium est choisie dans le groupe comprenant les oxydes et les hydroxydes de zirconium cristallisés ou amorphes, les halogènures, les

alcoolates, les sels solubles de zirconium.

**18.** Procédé selon la revendication 14, caractérisé par le fait que la source commune d'oxyde d'étain et d'oxyde de silice est choisie parmi les verres à base de silice et d'étain et les gels mixtes et la source commune d'oxyde de zirconium et d'oxyde de silice est choisie parmi les verres à base de silice et de zirconium et les gels mixtes.

**19.** Procédé selon l'une des revendications 6 à 18, caractérisé par le fait que les ions fluorure sont ajoutés dans le mélange réactionnel sous la forme d'acide fluorhydrique, de fluorhydrates d'amines ou d'ammonium quaternaire, de composés hydrolysables libérant les anions fluorure.

**20.** Procédé selon la revendication 19, caractérisé par le fait que les composés hydrolysables sont des sels fluorés contenant du silicium ou de l'étain choisis dans le groupe comprenant les fluorures d'étain et les fluorures de silicium.

**21.** Composé cristallin contenant de la silice et un oxyde d'élément tétravalent caractérisé par le fait qu'il répond à la formule (IV) suivante :

$$(Si_{96-x}\,T'_x)O_{192},\ 4\pm1(S^+F^-) \qquad (IV)$$

dans ladite formule (IV):
- x est compris entre 0,1 et 5,0,
- $S^+$ symbolise le cation provenant de l'agent structurant,
- T′ symbolise un élément tétravalent choisi dans le groupe formé par l'étain et le zirconium.

**22.** Composé cristallin selon la revendication 21 caractérisé par le fait qu'il répond à la formule (IVa) suivante:

$$(Si_{96-x}\,Sn_x)O_{192},\ 4\pm1(S^+F^-) \qquad (IVa)$$

dans ladite formule (IVa):
- x est compris entre 0,1 et 3,0 environ, de préférence, entre 0,1 et 2,0,
- $S^+$ symbolise le cation provenant de l'agent structurant.

**23.** Composé cristallin selon la revendication 21 caractérisé par le fait qu'il répond à la formule (IVb) suivante :

$$(Si_{96-x}\,Zr_x)O_{192},\ 4\pm1(S^+F^-) \qquad (IVb)$$

dans ladite formule (IVb):
- x est compris entre 0,1 et 4,0 environ, de préférence, entre 0,1 et 2,0,
- $S^+$ symbolise le cation provenant de l'agent structurant.

**24.** Composé cristallin selon l'une des revendications 21 à 23 caractérisé par le fait que $S^+$ symbolise un cation résultant de l'amine de formule (II), un cation de type ammonium quaternaire de formule (III), ou ces mêmes composés dans lesquelles l'azote est remplacé par un atome de phosphore.

**25.** Composé cristallin selon l'une des revendications 21 à 24 caractérisé par le fait qu'il présente un diagramme de diffraction de rayons X défini dans le tableau I.

**26.** Procédé de préparation du composé cristallin contenant de la silice et un oxyde d'élément tétravalent décrit dans l'une des revendications 21 à 25 caractérisé par le fait qu'il est obtenu selon un procédé comprenant les étapes suivantes :

(i) la préparation d'un mélange réactionnel en milieu aqueux contenant au moins une source de silicium au degré d'oxydation + 4, une source d'élément tétravalent, des ions fluorure et un agent structurant avec des rapports molaires T′/(Si+T′) compris entre 0,001 et 0,20, F/(Si+T′) entre 0,02 et 6, agent structurant/(Si+T′) entre 0,002 et 4 ; le pH du mélange réactionnel étant compris entre 1,5 et 12,0, de préférence, entre 6,0 et 11,0.

(ii) la cristallisation du mélange réactionnel

(iii) la récupération du précipité cristallin.

**27.** Utilisation des zéolithes à base de silice et d'oxydes d'éléments tétravalents décrites dans l'une des revendications 1 à 5, dans un procédé d'hydroxylation de phénols et d'éthers de phénols, par le peroxyde d'hydrogène, en présence d'une quantité efficace desdites zéolithes.

**28.** Utilisation selon la revendication 27 caractérisée par le fait que le phénol ou l'éther de phénol répond à

la formule générale (V) :

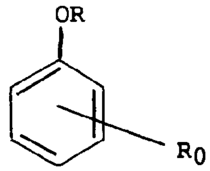

(V)

dans ladite formule (V):

– R représente un atome d'hydrogène, un groupement méthyle, un groupement éthyle ou un groupement phényle,

– $R_0$ représente un atome d'hydrogène, un radical alkyle ayant 1 à 4 atomes de carbone, un radical alkoxy ayant 1 à 4 atomes de carbone, un radical phényle ou cyclohexyle.

**29.** Utilisation selon la revendication 28, caractérisée par le fait par le fait que le phénol ou l'éther de phénol répond à la formule générale (V) dans laquelle R représente un atome d'hydrogène, un groupement méthyle ou un groupement éthyle, et $R_0$ représente un atome d'hydrogène, un groupement méthyle, éthyle ou tertiobutyle, un groupement méthoxy ou éthoxy.

**30.** Utilisation selon l'une des revendications 27 à 29 , caractérisée par le fait que le phénol ou l'éther de phénol est choisi parmi le phénol, l'anisole, l'orthocrésol, le métacrésol, le paracrésol, le tertiobutyl-4 phénol, le méthoxy-2 phénol, le méthoxy-4 phénol.

**31.** Utilisaton selon l'une des revendications 27 à 30, caractérisée par le fait que le catalyseur représente en poids par rapport au poids du composé de formule (V) engagé de 0,1 % à 20 % et de préférence de 0,5 % à 10 %.

Office européen des brevets

# RAPPORT DE RECHERCHE EUROPEENNE

Numero de la demande

EP 91 40 1717

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-3 941 871 (F.G. DWYER et al.)<br>* Exemples 1,7 *<br>--- | | C 01 B 33/20<br>B 01 J 29/04<br>C 07 C 37/60<br>C 07 C 41/26 |
| A | CHEMICAL ABSTRACTS, vol. 112, no. 26, 25 juin 1990, page 136, résumé no. 237640c, Columbus, Ohio, US; W. PANG et al.: "Preparation of tin-ZSM-5 type zeolite by gas-solid substitution reaction", & SHIYOU XUEBAO, SHIYOU JIAGONG 1989, 5(2), 93-8<br>--- | | |
| A | EP-A-0 077 523 (HOECHST AG)<br>--- | | |
| A | EP-A-0 346 250 (RHONE-POULENC CHIMIE)<br>----- | | |
| | | | **DOMAINES TECHNIQUES RECHERCHES (Int. Cl.5)** |
| | | | C 01 B<br>C 07 C |

Le présent rapport a été établi pour toutes les revendications

| Lieu de la recherche | Date d'achèvement de la recherche | Examinateur |
|---|---|---|
| LA HAYE | 08-10-1991 | BREBION J.CH. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)